# EUROPEAN PATENT APPLICATION

(11) **EP 4 050 029 A1**
(43) Date of publication of application: **31.08.2022**
(21) Application number: 21893100.4
(22) Date of filing: 22.12.2021
(51) Int. Cl.: C07K 16/28, A61K 39/395, A61P 35/00, A61P 35/02

(54) **ANTIBODY TARGETING CD47 AND APPLICATION THEREOF**

(30) Priority: 23.12.2020 CN 202011544262
(71) Applicant: Guangdong Feipeng Pharmaceutical Co., Ltd, Dongguan, Guangdong 523808 (CN)
(72) Inventor: HUO, Yongting, Dongguan, Guangdong 523808 (CN); LU, Lisheng, Dongguan, Guangdong 523808 (CN); TU, Jingjing, Dongguan, Guangdong 523808 (CN); LU, Di, Dongguan, Guangdong 523808 (CN); ZHANG, Chan, Dongguan, Guangdong 523808 (CN); YAN, Jiaqing, Dongguan, Guangdong 523808 (CN)
(74) Representative: Biggi, Cristina
(86) International application number: PCT/CN2021/140404
(87) International publication number: WO 2022/135460

(57) **Abstract**

The present application relates to the field of biomedicines, in particular to an antibody targeting CD47 and an application thereof. The anti-CD47 antibody provided by the present application may specifically bind to tumor cells, block the signal of human SIRPα and human CD47, and promote the phagocytosis of the tumor cells by macrophages. The antibody has high affinity, strong specificity, and good safety. The red blood cell (RBC) agglutination is not caused, and it shows very weak binding to RBC and platelets.

## Description

### Cross-Reference to Related Application

The present invention claims priority to Chinese patent application CN 202011544262.6, filed to the China National Intellectual Property Administration on December 23, 2020 and entitled "Antibody Targeting CD47 and Application thereof", the invention of which is hereby incorporated by reference in its entirety.

### Technical Field

The present invention belongs to the technical field of biological medicines. Specifically, it relates to an antibody targeting CD47 and an application thereof, and more specifically, relates to an anti-CD47 antibody or an antigen-binding fragment thereof, a nucleic acid, a vector, a cell, a composition, an application and a kit.

### Background

Cancer immunotherapy is a major event in the field of biological sciences in recent years. Immune checkpoint inhibitor therapies of T-cell-based CTLA4 antibody, PD-1 antibody, PD-L1 antibody and the like and cell therapies of CAR-T, TCR-T and the like are all hot immunotherapies in recent years. These revolve around how to restore T cell functions without exception, and in other words, how to improve the acquired immune system capacity. However, a road of using an immune checkpoint as a target point, and activating the T cell function, to improve the capacity of an acquired immune system so as to conquer a cancer is still full of twists and turns. However, the action of an innate immune system in tumor immunotherapy is not exerted for a long time. In fact, in the entire tumor-infiltrating area, macrophages account for about 50% of tumor tissues. More importantly, the number of the macrophages is inversely related to the prognosis of tumors, which further indicates that the macrophages have very important effect in the tumors. The phagocytosis exerted by the macrophages requires two signals to act simultaneously: one is the activation of an "eat me" signal on the surface of a target cell, and the other is the inactivation of a "don't eat me" signal on the surface of the same target. The absence of any one signal is not sufficient to trigger the phagocytosis. It is indicated from more and more evidences that CD47 is a type of the "don't eat me" signal, and it inhibits the phagocytosis of the macrophages by mutually combining with a signal regulatory protein α (SIRPα) on the surface of the macrophages. Tumor cells may also escape the macrophage phagocytosis through the expression of CD47 (for example, referring to EP2242512 and related documents cited therein).

CD47, also known as an integrin-associated protein (IAP), is a 50 kDa membrane protein with an amino-terminal immunoglobulin domain and a carboxy-terminal multiple transmembrane domain. It interacts with a plurality of ligands, including but not limited to SIRPα, SIRPγ, an integrin and thrombospondin-1 (TSP-1). SIRPα is mainly expressed on myeloid cells, including a macrophage, a myeloid dendritic cell (DC), a granulocyte, a mast cell and a precursor thereof, and including a hematopoietic stem cell. CD47/SIRPα interaction transmits the "don't eat me" signal, and prevents autophagy. The analysis of patient tumors and matched adjacent normal (non-tumor) tissues shows that the CD47 protein is overexpressed on cancer cells, and this effectively helps them escape innate immune surveillance and elimination. Blocking the interaction of CD47-SIRPα with an anti-CD47 antibody has already shown to effectively induce the phagocytosis of the tumor cells in vitro and inhibit the growth of various hematological and solid tumors in vivo. Therefore, CD47 is an effective target for cancer therapy, and an appropriate antagonist thereof is required to prepare human therapeutic agents.

### Summary

A CD47 monoclonal antibody has the higher targeting binding property to red blood cells, and it is easy to cause the red blood cell agglutination, so that the therapeutic effect of the corresponding antibody is greatly reduced, and drug side effects are caused. The present invention aims to provide an antibody targeting CD47, and the antibody may block the binding of CD47 to SIRPα, and promote the phagocytosis of macrophages. What is more commendable is that it may prevent red blood cell blood from agglutinating to a certain extent, and the safety is good.

A purpose of the present invention is to provide an anti-CD47 antibody or an antigen-binding fragment thereof, and the antibody contains the following complementarity determining regions (CDRs):
LCDR1 as shown in SEQ ID NO:1 or an amino acid sequence having at least 95% of sequence identity with SEQ ID NO:1, LCDR2 as shown in SEQ ID NO:2 or an amino acid sequence having at least 95% of sequence identity with SEQ ID NO:2, and LCDR3 as shown in SEQ ID NO:3 or an amino acid sequence having at least 95% of sequence identity with SEQ ID NO:3; and
HCDR1 as shown in SEQ ID NO:10 or an amino acid sequence having at least 95% of sequence identity with SEQ ID NO:10, HCDR2 as shown in SEQ ID NO:11 or an amino acid sequence having at least 95% of sequence identity with SEQ ID NO:11, and HCDR3 as shown in SEQ ID NO:12 or an amino acid sequence having at least 95% of sequence identity with SEQ ID NO:12.

Another purpose of the present invention is to provide a nucleic acid, a vector, a cell or a pharmaceutical composition related to the anti-CD47 antibody or the antigen-binding fragment thereof.

The present invention further relates to an application of the anti-CD47 antibody or the antigen-binding fragment thereof, and the related nucleic acid, vector, cell or pharmaceutical composition in preparation of a drug for treating and/or preventing a CD47-positive tumor.

The present invention further relates to an application of the anti-CD47 antibody or the antigen-binding fragment thereof, and the related nucleic acid, vector or cell in preparation of a kit for detecting CD47 or diagnosing a CD47-related disease.

The present invention further provides a kit for detecting CD47, and the kit contains the anti-CD47 antibody or the antigen-binding fragment thereof.

The present invention further provides a kit for diagnosing a CD47-related disease, and the kit contains the anti-CD47 antibody or the antigen-binding fragment thereof.

### Brief Description of the Drawings

Fig. 1 is the mean fluorescence intensity of a CD47 antibody (7A11H11, 7A11H12, 7A11H22, 7A11H32, 7A11H42, positive control antibody Hu5F9-G4 and hIgG4-isotype control) binding to human CD47.
Fig. 2 is the mean fluorescence intensity of a CD47 antibody (7A11H52, 7A11H14, 7A11H15, 7A11H33, 7A11H34, 7A11H35, 7A11H55, positive control antibody Hu5F9-G4 and hIgG4-isotype control) binding to human CD47.
Fig. 3 is the mean fluorescence intensity of a CD47 antibody (7A11H11, 7A11H12, 7A11H22, 7A11H32, 7A11H42, 7A11H52, positive control antibody Hu5F9-G4 and hIgG4-isotype control) binding to monkey CD47.
Fig. 4 is the mean fluorescence intensity of a CD47 antibody (7A11H14, 7A11H15, 7A11H33, 7A11H34, 7A11H35, 7A11H55, positive control antibody Hu5F9-G4 and hIgG4-isotype control) binding to monkey CD47.
Fig. 5 is a result of CD47/SIRPα binding inhibition of human CD47 by a CD47 antibody (7A11H11, 7A11H12, 7A11H22, 7A11H32, 7A11H42 and hIgG4-isotype control).
Fig. 6 is a result of CD47/SIRPα binding inhibition of human CD47 by a CD47 antibody (7A11H52, 7A11H14, 7A11H15, 7A11H33, 7A11H34, 7A11H35, and 7A11H55).
Fig. 7 is a result of ability testing of the CD47 antibody to promote macrophage phagocytosis of a tumor cell.
Fig. 8 is a result of red blood cell (RBC) agglutination ability testing of the CD47 antibody.
Fig. 9 is a testing result of binding ability of the CD47 antibody to a human red blood cell.
Fig. 10 is an analysis result of binding of the CD47 antibody to a human platelet.
Fig. 11 is an analysis result of an effect of the CD47 antibody on phagocytosis of a red blood cell by an activated macrophage.
Fig. 12 is an anti-tumor result of the CD47 antibody on a human B lymphocyte subcutaneous xenograft model.
Fig. 13 is an anti-tumor result of the CD47 antibody on a human malignant melanoma model.

### Detailed Description of the Embodiments

The present invention is further described below in combination with the specific embodiments, but the embodiments do not limit the present invention in any form. Unless otherwise specified, reagents, methods and devices used in the present invention are conventional reagents, methods and devices in the technical field.

Unless otherwise specified, the reagents and materials used in the following embodiments are commercially available.

The present invention relates to an anti-CD47 antibody or an antigen-binding fragment thereof, and the antibody contains the following CDRs:
LCDR1 as shown in SEQ ID NO:1 or an amino acid sequence having at least 95% of sequence identity with SEQ ID NO:1, LCDR2 as shown in SEQ ID NO:2 or an amino acid sequence having at least 95% of sequence identity with SEQ ID NO:2, and LCDR3 as shown in SEQ ID NO:3 or an amino acid sequence having at least 95% of sequence identity with SEQ ID NO:3; and
HCDR1 as shown in SEQ ID NO:10 or an amino acid sequence having at least 95% of sequence identity with SEQ ID NO:10, HCDR2 as shown in SEQ ID NO:11 or an amino acid sequence having at least 95% of sequence identity with SEQ ID NO:11, and HCDR3 as shown in SEQ ID NO:12 or an amino acid sequence having at least 95% of sequence identity with SEQ ID NO:12.

An important advantage of the antibody or the antigen-binding fragment thereof is its high affinity to CD47.

An important advantage of the antibody or the antigen-binding fragment thereof is that it has the activity of blocking the binding of CD47 to SIRPα.

An important advantage of the antibody or the antigen-binding fragment thereof is its ability to promote a macrophage to phagocytize a tumor cell.

An important advantage of the antibody or the antigen-binding fragment thereof is that it has the effect of significantly reducing agglomeration of red blood cell blood.

An important advantage of the antibody or the antigen-binding fragment thereof is that it shows a very weak level of low or no binding to a red blood cell.

Because of the above properties, the antibody or the antigen-binding fragment thereof may preferably be used as an antibody drug.

In the present invention, a technical term "antibody or antigen-binding fragment thereof" is a protein that binds to a specific antigen, and it generally refers to all proteins and protein fragments containing CDRs. "Antibody" specifically refers to a full-length antibody. A term "full-length antibody" includes a polyclonal antibody and a monoclonal antibody.

A term "antigen-binding fragment" is a substance containing a part or all of CDRs of an antibody, and it lacks at least some of amino acids present in a full-length chain but is still capable of specifically binding to an antigen. This type of the fragment has the biological activity, because it binds to a target antigen, and may compete with other antigen-binding molecules (including a complete antibody) for binding to a given epitope. In some embodiments, the antigen-binding fragment has a function of specifically recognizing and binding to CD47. In some embodiments, the antigen-binding fragment is a fragment which has a function of blocking the binding of CD47 to its ligand, and activating an immune cell, and in one aspect, this type of the fragment is selected from Fab (consisting of a complete light chain and Fd), Fv (consisting of VH and VL), ScFv (a single-chain antibody, VH and VL are linked by a linker peptide) or a single-domain antibody (consisting of VH only). The fragment may be generated by a recombinant nucleic acid technology, or may be generated by enzymatic lysis or chemical cleavage of the antigen-binding molecules (including the complete antibody).

A term "complementarity determining region" or "CDR" refers to a hypervariable region of a heavy chain and a light chain of an immunoglobulin. There are three heavy chain CDRs and three light chain CDRs. Here, depending on the situation, terms "CDR" and "CDRs" are used to refer to a region containing one or more or even all of major amino acid residues that contribute to the binding affinity of the antibody or the antigen-binding fragment thereof to an antigen or an epitope recognized by it. In another specific embodiment, a CDR region or CDR refers to the hypervariable region of the heavy chain and the light chain of the immunoglobulin defined by the international immunogenetics information system (IMGT).

In the present invention, the complementarity determining region of the heavy chain is represented by HCDR, and the complementarity determining region of the light chain is represented by LCDR. Common CDR marking methods used in the field include: a Kabat numbering scheme, a Chothia and Lesk numbering scheme, and a new standardized numbering system introduced by Lefranc et al. in 1997 for all protein sequences of an immunoglobulin superfamily. Kabat et al. were the first to propose a standardized numbering scheme for an immunoglobulin variable region. In their compilation of "Sequences of Proteins of Immunological Interest", the amino acid sequences of light chain (λ, κ) variable regions and antibody heavy chains, as well as the variable regions of T cell receptors (α, β, γ, and δ) are aligned and numbered. In the past few decades, the accumulation of sequences has led to the creation of a KABATMAN database, and the Kabat numbering scheme is generally considered as a widely adopted standard for numbering the antibody residues. The present invention adopts a Kabat annotation standard to mark the CDR regions, but the CDR regions marked by other methods also belong to the protection scope of the present invention.

Terms "specific recognizing", "selective binding", "selectively binding" and "specifically binding" or the like refer to the binding of the antibody or the antigen-binding fragment thereof to a predetermined epitope on an antigen. Typically, the antibody or the antigen-binding fragment thereof binds with an affinity (K_{D}) of less than about 10⁻⁶M, for example, less than about 10⁻⁷ M, 10⁻⁸ M, 10⁻⁹M, or 10⁻¹⁰ M or less.

In the present invention, an object specifically recognized by the provided antibody or antigen-binding fragment thereof may be CD47 derived from various genera, such as human, mouse, and monkey (such as, cynomolgus monkey).

In some embodiments, the antibody contains at least one of a heavy chain variable region sequence and a light chain variable region sequence, at least a part of at least one of the heavy chain variable region sequence and the light chain variable region sequence is from at least one of a mouse-derived antibody, a humanized antibody, a primate-derived antibody or a mutant thereof.

In some embodiments, the amino acid sequence of the light chain variable region is as shown in any one of SEQ ID NO:5~9 or a sequence having at least 95% of the identity with any one of SEQ ID NO:5~9.

In some embodiments, the amino acid sequence of the heavy chain variable region is as shown in any one of SEQ ID NO:14~18 or a sequence having at least 95% of the identity with any one of SEQ ID NO:14~18.

In some embodiments, the amino acid sequence of the light chain variable region is as shown in SEQ ID NO:5, the amino acid sequence of the heavy chain variable region is as shown in SEQ ID NO:14; or the amino acid sequence of the light chain variable region is as shown in SEQ ID NO:6, and the amino acid sequence of the heavy chain variable region is as shown in SEQ ID NO:14; or the amino acid sequence of the light chain variable region is as shown in SEQ ID NO:6, and the amino acid sequence of the heavy chain variable region is as shown in SEQ ID NO:15; or the amino acid sequence of the light chain variable region is as shown in SEQ ID NO:6, and the amino acid sequence of the heavy chain variable region is as shown in SEQ ID NO:16; or the amino acid sequence of the light chain variable region is as shown in SEQ ID NO:6, and the amino acid sequence of the heavy chain variable region is as shown in SEQ ID NO:17; or the amino acid sequence of the light chain variable region is as shown in SEQ ID NO:6, and the amino acid sequence of the heavy chain variable region is as shown in SEQ ID NO:18; or the amino acid sequence of the light chain variable region is as shown in SEQ ID NO:8, and the amino acid sequence of the heavy chain variable region is as shown in SEQ ID NO:14; or the amino acid sequence of the light chain variable region is as shown in SEQ ID NO:9, and the amino acid sequence of the heavy chain variable region is as shown in SEQ ID NO:14; or the amino acid sequence of the light chain variable region is as shown in SEQ ID NO:7, and the amino acid sequence of the heavy chain variable region is as shown in SEQ ID NO:16; or the amino acid sequence of the light chain variable region is as shown in SEQ ID NO:8, and the amino acid sequence of the heavy chain variable region is as shown in SEQ ID NO:16; or the amino acid sequence of the light chain variable region is as shown in SEQ ID NO:9, and the amino acid sequence of the heavy chain variable region is as shown in SEQ ID NO:16; or the amino acid sequence of the light chain variable region is as shown in SEQ ID NO:9, and the amino acid sequence of the heavy chain variable region is as shown in SEQ ID NO:18.

In some embodiments, the antibody has a constant region, and the heavy chain constant region is selected from any one of IgGI, IgG2, IgG3, IgG4, IgA, IgM, IgE or IgD; and the light chain constant region is a κ or λ chain.

In some embodiments, the species source of the constant region is selected from mouse, rabbit, sheep, monkey, or human.

In some embodiments, the antibody is any one or more of a CDR-transplanted antibody, a multimeric antibody or a bispecific antibody.

In some embodiments, the antigen-binding fragment is any one or more of F (ab') ₂, Fab, scFv, Fv, and a single-domain antibody.

In some embodiments, the CD47 is human CD47, mouse CD47 or monkey CD47.

The present invention further relates to a nucleic acid, and the nucleic acid encodes the anti-CD47 antibody or the antigen-binding fragment thereof.

The nucleic acid is usually a ribonucleic acid (RNA) or a deoxyribonucleic acid (DNA), and a nucleic acid molecule may be single-stranded or double-stranded, but is preferably a double-stranded DNA. The nucleic acid is "operably linked" while it is placed in a functional relationship with another nucleic acid sequence. For example, if a promoter or an enhancer affects the transcription of a coding sequence, the promoter or the enhancer is operably linked to the coding sequence. The DNA nucleic acid is preferably used while it is linked into a vector.

Furthermore, since the antibody is a membrane protein, the nucleic acid generally carries a signal peptide sequence.

The present invention further relates to a vector, and the vector carries the above nucleic acid.

A term "vector" refers to a nucleic acid delivery tool into which a polynucleotide may be inserted. While the vector may express the protein encoded by the inserted polynucleotide, the vector is called as an expression vector. The vector may be introduced into a host cell by transformation, transduction or transfection, so that a genetic material element carried by it may be expressed in the host cell. The vector is well-known to those skilled in the art and includes, but is not limited to: a plasmid; a phagemid; a cosmid; an artificial chromosome, such as a yeast artificial chromosome (YAC), a bacterial artificial chromosome (BAC) or a P1-derived artificial chromosome (PAC); and a phage such as a λ phage or an M13 phage and an animal virus. The animal virus that may be used as the vector includes, but is not limited to, a retrovirus (including a lentivirus), an adenovirus, an adeno-associated virus, a herpesvirus (such as, a herpes simplex virus), a poxvirus, a baculovirus, a papillomavirus, and a papovavirus (such as SV40). In some embodiments, the vector described in the present invention contains a regulatory element commonly used in genetic engineering, such as the enhancer, the promoter, an internal ribosome entry site (IRES) and other expression control elements (for example, a transcription termination signal, or a polyadenylation signal and a polymeric U sequence).

The present invention further relates to a cell, and the cell carries the above nucleic acid or contains the above vector. The nucleic acid encoding the anti-CD47 antibody or the antigen-binding fragment thereof is used to link to the vector, then expressed in the cell and the corresponding antibody may be obtained. The vector may be introduced into a eukaryotic cell, especially a mammalian cell, to construct and obtain the cells capable of expressing the anti-CD47 antibody or the antigen-binding fragment thereof described in the present invention.

As used herein, expressions of "cell", "cell line" and "cell culture" are used interchangeably, and all such names include a progeny. Thus, words "transformant" and "transformed cell" include a primary test cell and a culture derived therefrom, regardless of the number of transfers. It should also be understood that, due to deliberate or unintentional mutations, all progenies may not be exactly the same in terms of DNA content. A mutant progeny that has the same function or biological activity as screened in the original transformed cell are included. In the case that a different name is meant, it is clear from the context.

The present invention further relates to a pharmaceutical composition, and the pharmaceutical composition contains the anti-CD47 antibody or the antigen-binding fragment thereof, the nucleic acid, the vector or the cell.

In some embodiments, the composition further contains a pharmaceutically acceptable carrier. The "pharmaceutically acceptable carrier" may include any and all solvents, dispersion media, coatings, antibacterial and antifungal agents, isotonic agents and absorption delaying agents and the like that are physiologically compatible, to prolong the shelf life or efficacy of the antibody.

An application of the anti-CD47 antibody or the antigen-binding fragment thereof, the nucleic acid, the vector, the cell or the composition in preparation of a drug for treating and/or preventing a CD47-positive tumor should also be within the protection scope of the present invention.

The drug may reduce the red blood cell blood agglutination and reduce its binding activity with the red blood cells.

An application of the anti-CD47 antibody or the antigen-binding fragment thereof, the nucleic acid, the vector or the cell in preparation of a kit for detecting CD47 or diagnosing a CD47-related disease should also be within the protection scope of the present invention.

The present invention further provides a kit for detecting CD47, and the kit contains the anti-CD47 antibody or the antigen-binding fragment thereof.

The present invention further provides a kit for diagnosing a CD47-related disease, and the kit contains the anti-CD47 antibody or the antigen-binding fragment thereof.

The present invention has the following beneficial effects.

The present invention provides the anti-CD47 antibody or the antigen-binding fragment thereof. The antibody does not agglutinate the red blood cell in vitro, and more commendably, it shows the very weak level of low or no binding to the red blood cell; it may effectively block the binding of CD47 to SIRPα, and activate and mediate the phagocytic activity of the macrophages on the tumor cells, it shows the significant targeting specificity with the CD47-positive tumor cell, is high in affinity, and strong in specificity, without causing the red blood cell agglutination, and shows the extremely weak binding to human red blood cells and platelets, the safety is good; and therefore, the anti-CD47 antibody or the antigen-binding fragment thereof may be used as a very promising target site in a tumor immunotherapy system, play a strong role in human tumor treatment, and have a broad application prospect in preparation of the drug for treating the CD47-positive tumors.

Embodiments of the present invention are described in detail below in combination with the embodiments.

### Example 1: Screening of anti-CD47 mouse monoclonal antibody

### 1. Construction of hCD47-ECD-HIS recombinant expression plasmid

The DNA sequence encoding human CD47 extracellular region (hCD47-ECD) following by 6 ×HIS tag sequence was synthesized based on a template (CEJ 94640.1) from GenBank, and then was cloned into an expression vector pCDNA3.4 (Thermo Company), to establish a recombinant eukaryotic expression plasmid of a CD47 extracellular full-length protein, named hCD47-ECD-HIS recombinant plasmid DNA.

### 2. Expression and purification of hCD47-ECD-HIS recombinant protein

The expression and purification of the hCD47-ECD-HIS recombinant protein includes the following steps.
(1) On the day before transient transfection, Expi293 (ThermoFisher Scientific; A14635) cells were passaged, inoculated with a Dynamis medium (gibco; A2617502) at a density of 2*10⁶ in a 1 L shake flask (conning; 431147), and placed in a cell culture shaker (Adolf Kuhner; ISF4-XC) and cultured at 37°C, 8% CO₂, and 120 rpm.
   On the day of transfection, the Expi293 cells were counted with a cell counter (Countstar; IC1000), and the cell density was diluted and adjusted to 2.9*10⁶ with fresh Dynamis culture solution; it was ready for the transfection; PEI:DNA = 3:1; mixed uniformly for 5 min, the two were gently mixed for 20 times and standing for 15~30 min; a DNA-PEI mixture was added to the Expi293 cells, mixed uniformly, and placed in the cell culture shaker (Adolf Kuhner; ISF4-XC) and cultured at 37°C, 8% CO₂, and 120 rpm; and after 4 h of the transfection, a double-antibody (gibco; 15140122) and an anticoagulant (gibco; 0010057) were supplemented.
(2) Supernatant harvesting: the transfection is continuously cultured for 7 days, and then a sample was collected. Firstly it was centrifuged at a low speed of 1000 rpm × 10 min at 4°C, and then centrifuged at a high speed of 12000 rpm × 30 min at 4°C; and the cell culture supernatant was collected, and filtered using 0.22 um filter.
(3) Purification by HisTrap affinity chromatography column: the supernatant was loaded on the HisTrap affinity chromatography column at a speed of 1 mL/min. After loading was completed, the chromatography column was washed with 5 column volumes of 20 mM Tris-HCl, and 150 mM NaCl pH 8.0 equilibrium solution; and the chromatography column was washed with 5 column volumes of 20 mM Tris-HCl, 150 mM NaCl, and 0~500 mM imidazole pH 8.0 eluent, and the eluting peak was collected. The purified CD47-ECD protein was identified by polyacrylamide gel electrophoresis (SDS-PAGE).

### 3. Screening of anti-CD47 mouse monoclonal antibody

The hCD47-ECD-HIS recombinant protein (hereinafter referred to as an hCD47 antigen) purified in the step 2 was used for immunization of BALB/C mice (purchased from the Guangdong Experimental Animal Center). A specific method is as follows.
(1) Animal immunization: the purified hCD47 antigen was emulsified with a complete Freund's adjuvant, the BALB/C mice with 6~8 weeks old were immunized with a subcutaneous or intraperitoneal injection method, the immunization dose was 25 µg/mouse, and there were 5 mice in each group; the second immunization was performed after 1 week, it was emulsified with an incomplete Freund's adjuvant, and the immunization dose was 25 µg/mouse. After 4 times of the immunization, tail blood was collected and diluted in a gradient by an enzyme-linked immunosorbent assay (ELISA) to determine a serum titer; and the immunization was reinforced three days before fusion, and the mouse with the highest antibody titer was selected for cell fusion.
(2) Cell fusion: BALB/C-derived sp2/0 (ATCC^{®} CRL-1581) was used as myeloma cells, and was in a logarithmic growth phase while fusion; a spleen of the immunized mouse was taken to prepare single-cell suspension of lymphocytes; the mouse spleen lymphocytes and myeloma cells were mixed at a ratio of 1:5~1:10, and 1 mL of 50% PEG1500 (pH 8.0) at 37°C was dropwise added, and an incomplete medium DMEM and other stop solution were added. A HAT medium was suspended and mixed uniformly after the supernatant was discarded through centrifuging, plated in a 96-well plate, placed and cultured in a constant temperature incubator at 37°C and 5% CO₂. After one week of culture, the first solution exchange was performed with an HT medium, and after another three days of the culture, the second solution exchange was performed with the HT medium.
(3) Screening and cloning: after 2 weeks of the fusion, a cell supernatant was taken for an ELISA test, to detect a binding situation of the cell supernatant to the purified CD47-ECD-His recombinant protein, and after cells of which ELISA results were positive were screened out, the second ELISA test was performed to retest; and the cells in the positive wells were subjected to limited dilution, and an ELISA value was determined 7 days after each limited dilution, and the monoclonal wells with the higher OD280 positive value were selected for the limited dilution, until the ELISA results of the entire 96-well plate were positive. Monoclonal strains with the high positive values were picked.
(4) Preparation and purification of cell supernatant monoclonal antibody: positive clones were cultured in a T25 culture flask with the DMEM medium containing 15% serum, and while enlarging the culture, they were centrifuged at 800 rpm/min for 5 min, the supernatant was discarded and cells were transferred into a 500 mL shake flask, a serum-free medium (Hybridoma-SFM Complete DPM; Gibco; 12300-067) was added, so that the cell density was about 3×10⁵ cells/mL. After being continuously cultured for 1∼2 weeks, while the cell death rate reaches 60%~70%, cell suspension was collected, and centrifuged at a high speed of 8000 rpm/min for 20 min, the supernatant was taken, the supernatant was purified by an affinity chromatography, and the antibody was purified by a Protein A affinity chromatography. Sample loading; flow-washing: flow-washing with 2.5 M phosphate buffered solution (PBS) with pH 7.4, until the UV280 baseline to be 0; elution: eluting with 0.1 M citric acid solution with pH 3.5, an elution tube was collected in each section of 2 mL, and each tube was added with 100 µL of 1 M Tris solution; the collected solution was concentrated; the concentration of the purified monoclonal antibody was determined, and the purity of the antibody was verified by SDS-PAGE gel; and it was sub-packaged (100 µL/tube), and stored at -80°C.

### Example 2: Humanized design and combination of mouse monoclonal antibody

Amino acid sequences of mouse antibody light chain CDRs are shown in SEQ ID NOs:1~3:

| | | |
|---|---|---|
| 7A11D3D3-LCDR1 | 7A11D3D3-LCDR2 | 7A11D3D3-LCDR3 |
| SEQ ID NO:1 | SEQ ID NO:2 | SEQ ID NO:3 |
| KSSQSLLNSRTRKNYLA | WASTRES | KQSYNLRT |

Amino acid sequences of mouse antibody heavy chain CDRs are shown in SEQ ID NOs:10~12:

| | | |
|---|---|---|
| 7A11D3D3-HCDR1 | 7A11D3D3-HCDR2 | 7A11D3D3-HCDR3 |
| SEQ ID NO:10 | SEQ ID NO:11 | SEQ ID NO:12 |
| SNWMN | MIHPSDSETRLNQKFKD | GTTVVDAFAY |

An amino acid sequence of a mouse antibody light chain variable region (>7A11D3D3-VL) was shown in SEQ ID NO:4.

An amino acid sequence of a mouse antibody heavy chain variable region (>7A11D3D3-VH) was shown in SEQ ID NO:13.

The humanized design was performed on the mouse antibody, 5 humanized sequences, 7A11-L01~05 respectively, were designed for a 7A11D3D3 mouse monoclonal antibody light chain, and amino acid sequences of humanized light chain variable regions (7A11-L01~05) were shown in SEQ ID NOs:5~9; and 5 humanized sequences, 7A11-H01~05 respectively, were designed for a heavy chain, and amino acid sequences of humanized heavy chain variable regions (7A11-H01~05) were shown in SEQ ID NOs:14~18.

The above amino acid sequences were combined, and the obtained antibodies and the sequences corresponding to the heavy chain variable regions (VH) and light chain variable regions (VL) thereof were shown in Table 1.

**Table 1: Antibody and sequences corresponding to heavy chain variable region and light chain variable region thereof**

| Antibody name | VH | VL |
|---|---|---|
| 7A11H11 | SEQ ID NO:14 | SEQ ID NO:5 |
| 7A11H12 | SEQ ID NO:14 | SEQ ID NO:6 |
| 7A11H22 | SEQ ID NO:15 | SEQ ID NO:6 |
| 7A11H32 | SEQ ID NO:16 | SEQ ID NO:6 |
| 7A11H42 | SEQ ID NO:17 | SEQ ID NO:6 |
| 7A11H52 | SEQ ID NO:18 | SEQ ID NO:6 |
| 7A11H14 | SEQ ID NO:14 | SEQ ID NO:8 |
| 7A11H15 | SEQ ID NO:14 | SEQ ID NO:9 |
| 7A11H33 | SEQ ID NO:16 | SEQ ID NO:7 |
| 7A11H34 | SEQ ID NO:16 | SEQ ID NO:8 |
| 7A11H35 | SEQ ID NO:16 | SEQ ID NO:9 |
| 7A11H55 | SEQ ID NO:18 | SEQ ID NO:9 |

### Example 3: Production of CD47 antibody

A humanized variable domain was combined with a secretion signal and human κ and human FclgG4S228P constant domains, cloned into a mammalian expression system, and transfected into a 293 cell to generate humanized mAb. A humanized variant was expressed as a full-length IgG molecule, secreted into a culture medium and purified with the protein A.

Humanized antibody and positive control antibody Hu5F9-G4 (a Hu5F9-G4 sequence has already disclosed in the US patent US2015/0183874A1) were transiently expressed in Expi293 cells and purified.

For the transient expression of the antibody in the Expi293 cells, a vector pCDNA3.4 was used, and a heavy chain and a light chain of the antibody were firstly cloned into the separate pCDNA3.4 vector, a PEI (purchased from Polysciences) chemical transfection reagent was used, the pCDNA3.4 vectors with the heavy chain and the light chain of the antibody molecule were transferred into the Expi293 cells according to a chemical transfection method, and the transferred and cultured Expi293 cells were transiently transfected according to a scheme provided by a manufacturer.

On the day before transient transfection, the Expi293 (ThermoFisher Scientific; A14635) cells were passaged, inoculated with a Dynamis medium (gibco; A2617502) at a density of 2E6 in a 1 L shake flask (conning; 431147), and placed and cultured in a cell culture shaker (Adolf Kuhner; ISF4-XC) at 37°C, 8% CO₂ and 120 rpm.

On the day of transfection, the Expi293 cells were counted with a cell counter (Countstar; IC1000), and the cell density was diluted and adjusted to 2.9E6 with fresh Dynamis culture solution; it was ready for the transfection; PEI:DNA = 3:1 mixed uniformly for 5 min, the two were gently mixed for 20 times and standing for 15~30 min; the DNA-PEI mixture was added to the Expi293 cells, mixed uniformly, and placed in the cell culture shaker (Adolf Kuhner; ISF4-XC) and cultured at 37°C, 8% CO₂, and 120 rpm; and after 4 h of the transfection, a double-antibody (gibco; 15140122) and an anticoagulant (gibco; 0010057) were supplemented.

Purification of harvested supernatant: the transfection was continuously cultured for 7 days, and then the sample was collected. First it was centrifuged at a low speed of 1000 rpm, 10 min and 4°C (Xiangyi H2050R), and then centrifuged at a high speed of 12000 rpm, 30 min and 4°C; and a cell culture supernatant was collected, and filtered at 0.22 um. The culture supernatant was used in a Protein A Sepharose column (GE Healthcare). The column was washed with PBS, and eluted with an elution buffer (0.1 M sodium citrate buffer, pH 3.0) to remove the protein. A collected component was neutralized with 1 M Tris pH 9.0. Finally, the purified sample was dialyzed with PBS.

### Example 4: CD47 antibody affinity determination

### 1. Experimental method

An equilibrium dissociation constant (KD) of the antibody of the present invention binding to human CD47 (hCD47) was determined by a biofilm interferometry (ForteBio). ForteBio affinity determination was performed according to a method in the prior art (Estep, P et al., High throughput solution-based measurement of antibody-antigen affinity and epitope binning. MAbs; 2013.5 (2): p.270-8), and it was specifically as follows: a sensor was used to equilibrate offline for 30 minutes in an analyzing buffer and detected online for 60 seconds to establish a baseline. The purified antibody obtained in Example 3 was loaded online to an AHC sensor (ForteBio) for ForteBio affinity determination, and then the sensor with the loaded antibody was exposed in 100 nM of the CD47 antigen and acted for 5 minutes, then the sensor was transferred to the analyzing buffer for dissociation for 5 minutes and used for dissociation rate measurement. Kinetic analysis was performed with a 1:1 binding model.

### 2. Experimental result

CD47 antibody affinity determination results were shown in Table 2, and the results showed that the CD47 antibody has high affinity.

**Table 2: CD47 antibody affinity determination results**

| Antibody name | KD (M) | Kon (1/Ms) | Kd (1/s) |
|---|---|---|---|
| 7A11H11 | 5.90E-10 | 5.90E-10 | 5.90E-10 |
| 7A11H12 | 4.59E-10 | 4.59E-10 | 4.59E-10 |
| 7A11H14 | 5.62E-10 | 5.62E-10 | 5.62E-10 |
| 7A11H15 | 2.12E-10 | 1.48E+06 | 3.13E-04 |
| 7A11H35 | 8.81E-10 | 8.81E-10 | 8.81E-10 |
| 7A11H22 | 7.40E-10 | 7.40E-10 | 7.40E-10 |
| 7A11H33 | 3.60E-10 | 3.60E-10 | 3.60E-10 |
| 7A11H42 | 3.09E-10 | 3.09E-10 | 3.09E-10 |

### Example 5: Binding of CD47 antibody to human CD47

### 1. Experimental method

Binding of the CD47 antibody of the present invention to human CD47 was measured in a measurement method based on a flow cytometry. The specific steps were as follows.

A cell line CCRF-CEM (Cell Bank of Chinese Academy of Sciences, Shanghai) expressing human CD47 was used, which belongs to human acute lymphoblastic leukemia T lymphocytes. CCRF-CEM cells (0.1×10⁶ cells) were mixed with experimental antibodies (the CD47 antibody of the present invention and a Hu5F9-G4 antibody) at different concentrations (the highest concentration is 30ug/mL, it was three-fold dilution, and there were 10 concentrations in total) in PBS containing 3% bovine serum albumin (BSA) and incubated on ice for 30 minutes. Then, the cells were washed at least twice, a PE Goat anti human IgG Fc (1:500x dilution) fluorescent secondary antibody was prepared with a FCM buffer (1XPBS+3%BSA), added to a corresponding 96-well plate according to 100 µL/well, and incubated in a refrigerator at 4°C for 30 min. The 96-well plate was taken out, and centrifuged at 250 g for 5min, after a supernatant was carefully removed, the FCM buffer was added at 200 µL/well, and it was centrifuged again at 250 g for 5 min, the supernatant was carefully removed. The cells were washed at least twice and resuspended using 100 µL/well 1xPBS, and analyzed by the flow cytometry, and a concentration-dependent curve was fitted with GraphPad according to the mean fluorescence intensity (MFI) thereof. The Hu5F9-G4 antibody was used as a positive control antibody.

### 2. Experimental result

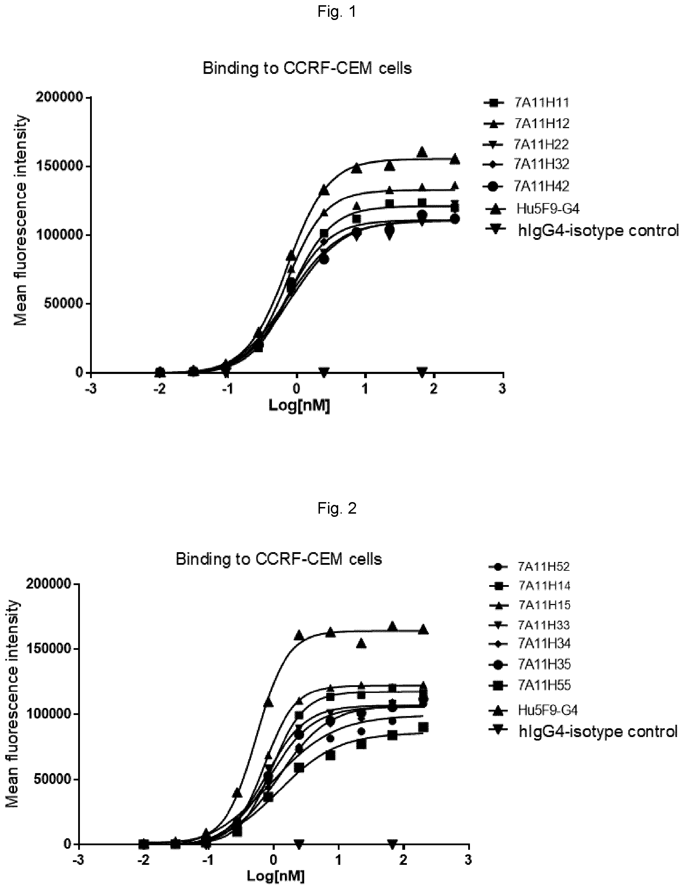

EC50 results of the CD47 antibody binding to human CD47 were shown in Table 3, the average fluorescence intensity of the CD47 antibody (7A11H11, 7A11H12, 7A11H22, 7A11H32, 7A11H42, positive control antibody Hu5F9-G4 and hIgG4-isotype control) binding to human CD47 was shown in Fig. 1, and the average fluorescence intensity of the CD47 antibody (7A11H52, 7A11H14, 7A11H15, 7A11H33, 7A11H34, 7A11H35, 7A11H55, positive control antibody Hu5F9-G4 and hIgG4-isotype control) binding to human CD47 was shown in Fig. 2. The results showed that the CD47 antibody of the present invention and the positive control antibody have comparable specific binding ability to human CD47 at a cellular level.

**Table 3: Determination results of binding ability of CD47 antibody to human CD47**

| Antibody name | EC50 (nM) |
|---|---|
| 7A11H11 | 0.8218 |
| 7A11H12 | 0.7157 |
| 7A11H22 | 0.8202 |
| 7A11H32 | 0.7172 |
| 7A11H42 | 0.7269 |
| 7A11H52 | 0.9148 |
| 7A11H14 | 0.9334 |
| 7A11H15 | 0.7234 |
| 7A11H33 | 0.7598 |
| 7A11H34 | 1.195 |
| 7A11H35 | 0.8623 |
| 7A11H55 | 1.209 |
| Hu5F9-G4 | 0.7289 |

### Example 6: Binding of CD47 antibody to monkey CD47

### 1. Experimental method

By transfecting a pCDNA3.4 vector carrying the full-length monkey CD47, a stable CHO cell line (CHO-cynoCD47 cell) overexpressing the monkey CD47 was generated, and the CHO-cynoCD47 cells (0.1×10⁶ cells) were mixed with experimental antibodies (the CD47 antibody of the present invention and a Hu5F9-G4 antibody) at different concentrations (the highest concentration is 10 ug/mL, it was three-fold dilution, and there were 10 concentrations in total) in PBS containing 3% BSA, and incubated on ice for 30 minutes. Then, the cells were washed at least twice, a PE Goat anti human IgG Fc (1:500x dilution) fluorescent secondary antibody was prepared with a FCM buffer (1XPBS+3%BSA), added to a corresponding 96-well plate according to 100 µL/well, and incubated in a refrigerator at 4°C for 30 min. The 96-well plate was taken out, and centrifuged at 250 g for 5min, after a supernatant was carefully removed, the FCM buffer was added at 200 µL/well, and it was centrifuged again at 250 g for 5 min, the supernatant was carefully removed. The cells were washed at least twice and resuspended with 1xPBS at 100 µL/well, and analyzed by the flow cytometry, and a concentration-dependent curve was fitted with GraphPad according to MFI thereof. The Hu5F9-G4 antibody was used as a positive control antibody.

### 2. Experimental result

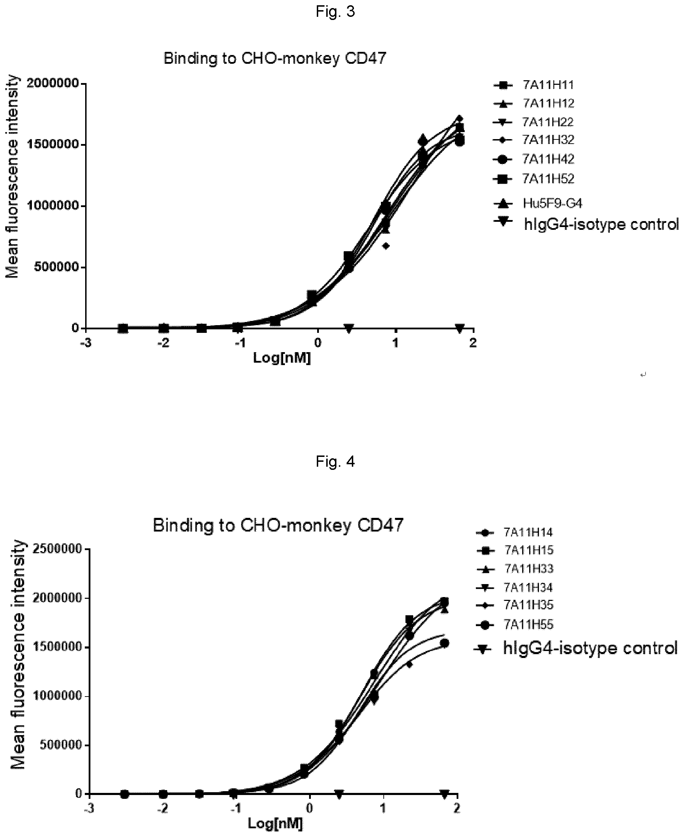

EC50 results of the CD47 antibody binding to monkey CD47 were shown in Table 4, the average fluorescence intensity of the CD47 antibody (7A11H11, 7A11H12, 7A11H22, 7A11H32, 7A11H42, 7A11H52, positive control antibody Hu5F9-G4 and hIgG4-isotype control) binding to monkey CD47 was shown in Fig. 3, and the average fluorescence intensity of the CD47 antibody (7A11H14, 7A11H15, 7A11H33, 7A11H34, 7A11H35, 7A11H55, positive control antibody Hu5F9-G4 and hIgG4-isotype control) binding to monkey CD47 was shown in Fig. 4. The results showed that in comparison, the antibody of the present invention and the positive control antibody have the comparable specific binding ability to human CD47 in the cellular level form.

**Table 4: EC50 results of CD47 antibody binding to monkey CD47**

| Antibody name | EC50 (nM) |
|---|---|
| 7A11H11 | 8.932 |
| 7A11H12 | 7.353 |
| 7A11H22 | 8.546 |
| 7A11H32 | 17.12 |
| 7A11H42 | 5 |
| 7A11H52 | 4.506 |
| 7A11H14 | 4.957 |
| 7A11H15 | 7.271 |
| 7A11H33 | 4.847 |
| 7A11H34 | 8.609 |
| 7A11H35 | 4.379 |
| 7A11H55 | 4.616 |
| Hu5F9-G4 | 5.123 |

### Example 7: CD47 antibody blocks the interaction of human CD47 ligand SIRPα with CD47

### 1. Experimental method

The ability of the CD47 antibody of the present invention to block the binding of human CD47 to SIRPα was determined by a flow cytometry. The specific steps were as follows:
Antibody dilution: an FCM buffer (1XPBS+3%BSA) was used to dilute the CD47 antibody of the present invention and the control antibody Hu5F9-G4 to 90ug/mL, then it was diluted to 10 concentrations in a 3-fold gradient, and a subtype control hlgG4 is diluted to 30 ug/mL, 1.1 ug/mL, and 0.04 ug/mL, and a ligand hSIRP α-mFC (AcroBiosystems) is diluted to 10ug/mL.
CCRF-CEM (Cell Bank of Chinese Academy of Sciences, Shanghai) cells were added to a 96-well V-type plate at 0.1 × 10⁶ cells/well, and hSIRPα-mFC binding was monitored under a condition of increasing the amount of the CD47 antibody. Bound SIRPα was determined with a PE Goat anti mouse IgG Fc secondary antibody (Biolegend). The Hu5F9-G4 antibody was used as a positive control antibody.

### 2. Experimental result

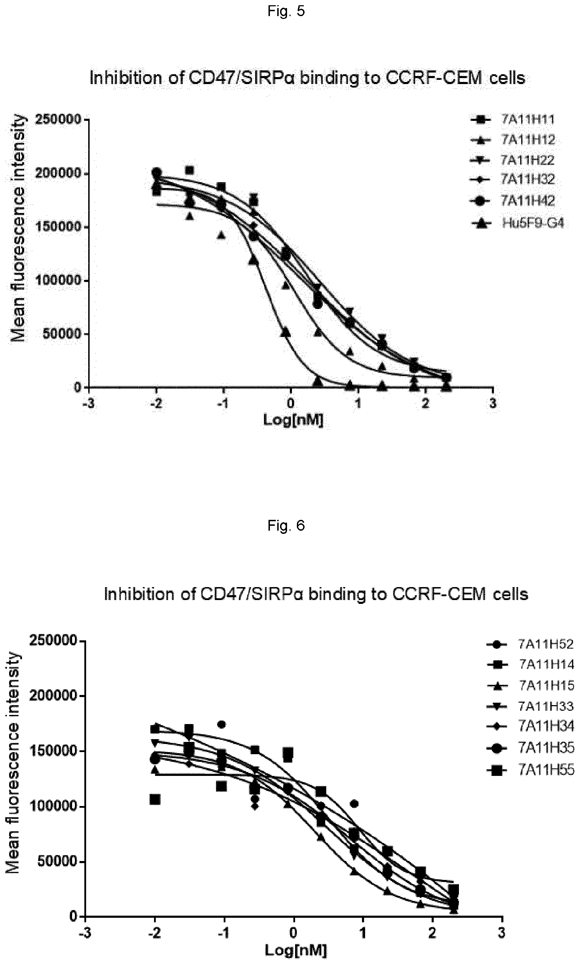

Results of CD47 antibody inhibition of CD47/SIRP α binding to human CD47 (7A11H11, 7A11H12, 7A11H22, 7A11H32, 7A11H42, Hu5F9-G4, and hIgG4-isotype control) were shown in Fig. 5, and Results of CD47 antibody inhibition of CD47/SIRPα binding to human CD47 (7A11H52, 7A11H14, 7A11H15, 7A11H33, 7A11H34, 7A11H35, and 7A11H55) were shown in Fig. 6. The results showed that the CD47 antibody of the present invention may significantly inhibit the binding of CD47 to SIRPα at a cellular level, and compared with the positive control antibody, the CD47 antibody of the present invention shows the comparable blocking ability.

### Example 8: Detection of CD47 antibody promoting ability of macrophage to phagocytose tumor cell

### 1. Experimental method

The ability of the CD47 antibody of the present invention to promote macrophage phagocytosis of tumor cells was measured in a measurement method based on a flow cytometry. The specific steps were as follows:
Fresh blood of a donor was taken and separated to obtain peripheral blood mononuclear cells (PBMC), and CD14-positive mononuclear cells were separated from PBMC by an hCD14 magnetic bead (Miltenyi/130-050-201). A complete medium containing rhGM-CSF (R&D; 7954-GM-010) was prepared, the final concentration of rhGM-CSF was 50 ng/mL, and the concentration of the CD14 positive mononuclear cells wais 5E5/mL, it was added to a cell culture dish at 20 mL/dish; it was transferred to a cell culture incubator at 5% CO₂ and 37°C, and the medium (containing 50 ng/mL GM-CSF) was replaced in half every 3 days; and it was continued to culture for 4 days. On the 8th day, the macrophage supernatant was aspirated into a 15 mL centrifuge tube, and pre-cooled DPBS was added at the same time, the cells were directly collected with a cell scraper.

The tumor cell line Jurkat (Cell Bank of Chinese Academy of Sciences, Shanghai) with high expression of human CD47 was selected as a target cell type, and the target tumor cells were fluorescently labeled according to instructions of a CellTraceTM CFSE kit. The labeled tumor cells were co-cultured with the above differentiated macrophages at a ratio of 1:1, and at the same time, the antibodies at final concentrations of 10 ug/mL, 1 ug/mL, and 0.1 ug/mL were added to incubate at 37°C for 2 hours. Then, the cells were washed at least twice, and carefully blown down, and allophycocyanin (APC) -labeled CD14 antibody (purchased from Biolegend; B259538) was added and incubated in PBS containing 0.1% BSA on ice (protected from light) for 30 minutes. The cells were washed at least twice and analyzed by a flow cytometry. The phagocytosed cell population was a population of cells that are CD14-positive in living cells and also positive for fluorescent dye CFSE (carboxyfluorescein diacetate, and succinimidyl ester). The Hu5F9-G4 antibody was used as a positive control antibody.

### 2. Experimental result

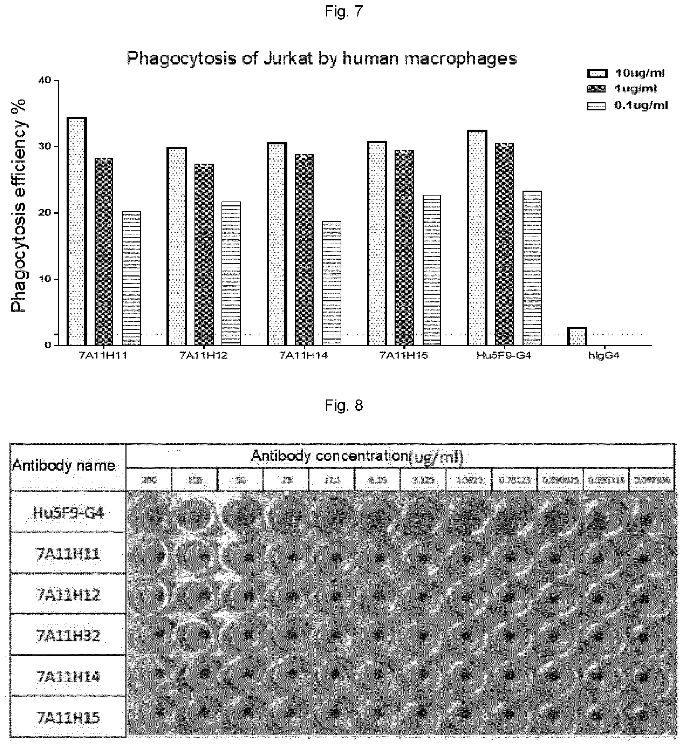

Measurement results of the ability of CD47 antibody to promote macrophage phagocytosis of tumor cells were shown in Fig. 7. The results showed that the CD47 antibody of the present invention has the ability to promote the macrophage phagocytosis of the tumor cells, and the ability to promote the macrophage phagocytosis of the tumor cells is comparable to the ability of the positive control antibody Hu5F9-G4.

### Example 9: Hemagglutination analysis of CD47 antibody to human red blood cell (RBC)

### 1. Experimental method

Hemagglutination analysis of RBC was performed to characterize the RBC agglutination ability of the CD47 antibody. The CD47 antibodies were screened for RBC agglutination by observing the ability of the antibodies to avoid sedimentation of human RBCs. A specific method was as follows.

RBCs were diluted to 2% in PBS, and the CD47 antibody (the concentration is 200 ug/mL, 100 ug/mL, 50 ug/mL, 25 ug/mL, 12.5 ug/mL, 6.25 ug/mL, 1.5625 ug/mL, 0.78125 ug/mL, 0.390625 ug/mL, 0.195313 ug/mL, and 0.097656 ug/mL successively) was dropwise added and incubated in a round-bottom 96-well plate at 37°C for 2 hours. The presence of unprecipitated RBCs was an evidence of RBC hemagglutination, and the unprecipitated RBCs were haze-like compared to the unagglomerated RBCs that form clear red dots. The Hu5F9-G4 antibody was used as a positive control antibody.

### 2. Experimental result

Measurement results of the RBC agglutination ability of the CD47 antibody were shown in Fig. 8. The results showed that while the concentration of the CD47 antibody reaches 200 ug/mL, no cell agglutination was caused, and it was indicated that the CD47 antibody of the present invention has the effect of significantly reducing RBC hemagglutination. In the treatment of cancers, side effects of the CD47 antibody may be significantly reduced, and the safety is good.

### Example 10: Binding analysis of CD47 antibody to human RBC

### 1. Experimental method

The CD47 monoclonal antibody has the property of binding to human RBC. For a CD47 antibody inhibitor, there is a potential risk of drug efficacy being interfered by RBCs and tumor off-target. If an antibody with low binding activity to RBC may be screened, the risk of off-target may be reduced, and its safety may be improved. Specific steps were as follows.
(1) Antibody dilution: an FACS buffer was used to dilute the CD47 antibody to an initial concentration of 20 µg/mL, the volume was 180 µL, it was diluted in a 3-fold gradient (60+120), and there were 11 concentrations.
(2) Cell counting and plating: RBC cells were centrifuged at 250 g for 5 min, then a supernatant was discarded, the cell density was adjusted to 2E+06 with the FACS buffer, and it was equally distributed into a 96-well V-type plate according to 100 µL/tube.
(3) The above diluted antibody was added to the cells at 100 µL/well, and incubated at 2°C ~8°C for 0.5 h.
(4) The 96-well plate was taken out, and centrifuged at 250 g for 5 min, after the supernatant was carefully removed, the FACS buffer was added at 200 µL/well, and it was centrifuged again at 250 g for 5 min, the supernatant was carefully removed.
(5) A PE goat anti-human IgG Fc (biolegend) fluorescent secondary antibody (1:500 dilution) was prepared with the FACS buffer, and added to a corresponding 96-well plate at 100 µL/well, the cells are resuspended, and incubated at 2°C~8°C for 30 min.
(6) The 96-well plate was taken out, and centrifuged at 250 g for 5 min, after a supernatant was carefully removed, the FACS buffer was added at 200 µL/well, and it was centrifuged again at 250 g for 5 min, the supernatant was carefully removed.
(7) It was resuspended with 1×PBS at 100 µL/well, and detected by FACS. Data was analyzed with a flow cytometer (Beckman, cytoflex), and plotted with GraphPad Prism. The Hu5F9-G4 antibody was used as a positive control antibody.

### 2. Experimental result

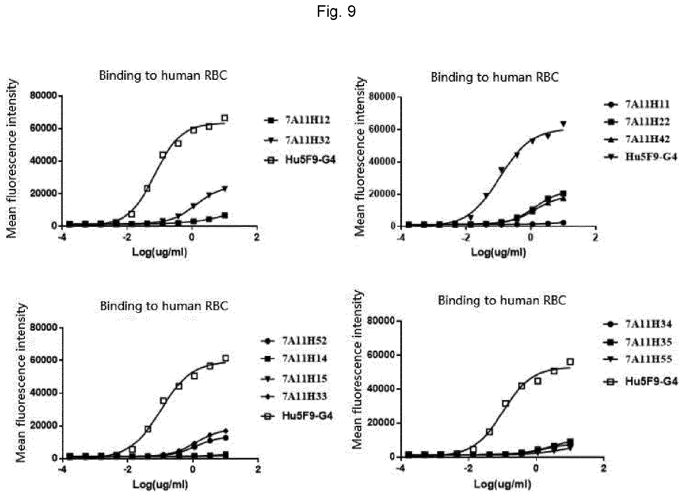

Measurement results of the binding ability of the CD47 antibody to human RBC were shown in Fig. 9. The results showed that compared with the positive control antibody, the binding activity of the CD47 antibody of the present invention to the human red blood cells is significantly reduced, and its safety as a drug is improved.

### Example 11: Analysis of CD47 antibody binding to platelet

### 1. Experimental method

Like the CD47 monoclonal antibody that binds to the human red blood cells, the CD47 monoclonal antibody has the active characteristics of binding to the platelets, and there are many side effects caused by reduction of the platelets. The antibody with low platelet binding may reduce the risk of off-target and improve its safety. A specific method was as follows.

Antibody dilution: the antibody was diluted to 20 µg/ml with a FACS buffer, and the volume was 240 µL.

Cell counting and plating: whole blood cells were diluted by 20 times, and equally distributed to a 96-well V-type plate according to 100 µL/tube.

The above diluted antibody was added to the cells at 100 µL/well, and incubated at 2°C-8°C for 0.5 h.

The 96-well plate was taken out, and centrifuged at 250 g for 5 min, after the supernatant was carefully removed, the FACS buffer was added at 200 µL/well, and it was centrifuged again at 250 g for 5 min, the supernatant was carefully removed.

A PE fluorescent secondary antibody (1:500 dilution) (PE goat anti-human IgG Fc; biolegend; 409304) was prepared with the FACS buffer, and added to a corresponding 96-well plate at 100 µL/well, and 1 µL of APC anti human CD61 (biolegend; 336411) was added to each well at the same time, the cells were resuspended, and incubated at 2°C -8°C for 30 min.

The 96-well plate was taken out, and centrifuged at 250 g for 5 min, after a supernatant was carefully removed, the FACS buffer was added at 200 µL/well, and it was centrifuged again at 250 g for 5 min, the supernatant was carefully removed.

It was resuspended with 1xPBS at 200 µL/well, and an FACS detection was performed. The Hu5F9-G4 antibody was used as a positive control antibody.

### 2. Experimental result

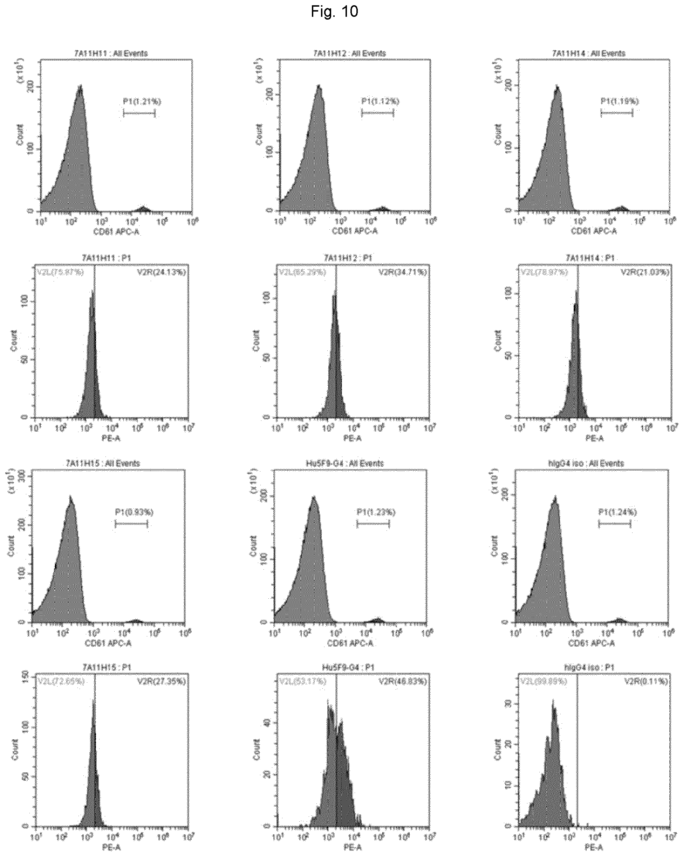

Binding analysis results of the CD47 antibody and platelets were shown in Fig. 10. The results showed that the CD47 antibody of the present invention has the significantly lower binding activity to the platelets than the positive control antibody and shows the better safety.

### Example 12: Analysis of effect of CD47 antibody on phagocytosis of RBC by activated macrophages

### 1. Experimental method

A detection method is the same as that of Example 8, the red blood cells were replaced with tumor cells as a target cell, and it was detected whether the CD47 antibody of the present invention has an activating effect on the phagocytosis of the red blood cells by the macrophages. The Hu5F9-G4 antibody was used as a positive control antibody.

### 2. Experimental result

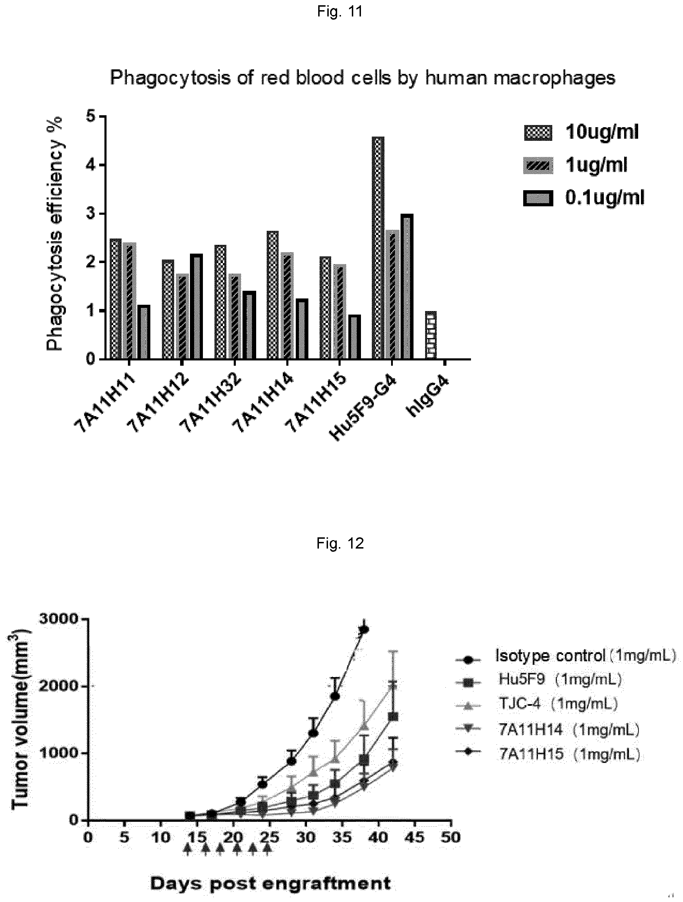

Results of the analysis of the effect of the CD47 antibody on the phagocytosis of the red blood cells by the activated macrophages were shown in Fig. 11. The results showed that the CD47 antibody of the present invention is very low in mediating the phagocytosis of the red blood cells by the macrophages, and the mediating effect is significantly lower than that of the positive control antibody and shows the better safety.

### Example 13: In vivo study of antitumor activity

### 1. Experimental method

70 NOD SCID female mice (purchased from Zhejiang Weitong Lihua Laboratory Animal Technology Co., Ltd.) were selected to construct a human B lymphocyte subcutaneous xenograft model (Raji) and a human malignant melanoma model (A375), and the antitumor activity of the CD47 antibody of the present invention is evaluated. In those studies, the Hu5F9-G4 antibody analog and TJC-4 antibody analog were used as positive control antibodies, and hlgG4 was used as an isotype control antibody.

### 2. Experimental result

Anti-tumor results of the CD47 antibody on the human B lymphocyte subcutaneous xenograft model were shown in Fig. 12, and the results showed that the CD47 antibody of the present invention is significantly better than the positive control antibody in the anti-human B lymphocyte subcutaneous xenograft effect.

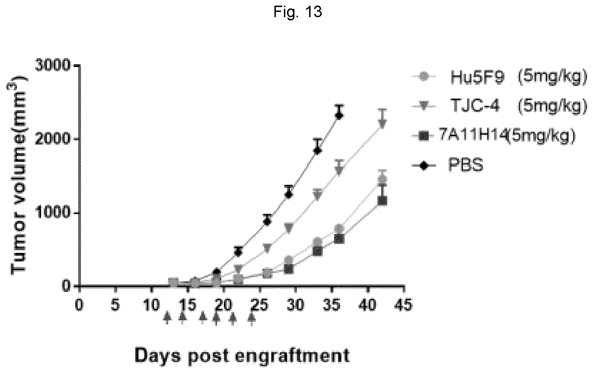

Anti-tumor results of the CD47 antibody on the human malignant melanoma model were shown in Fig. 13, and the results showed that compared with the positive control antibody, the CD47 antibody of the present invention is more advantageous in the anti-human malignant melanoma effect.

The above embodiments are preferred embodiments of the present invention, but the embodiments of the present invention are not limited by the above embodiments, and any other changes, modifications, replacements, combinations, simplifications made without departing from the spirit essence and principles of the present invention should be equivalent substitution modes and are all included in the protection scope of the present invention.

## Claims

1. An anti-CD47 antibody or an antigen-binding fragment thereof, wherein the antibody comprises the following complementarity determining regions (CDRs):
LCDR1 as shown in SEQ ID NO:1 or an amino acid sequence having at least 95% of sequence identity with SEQ ID NO:1, LCDR2 as shown in SEQ ID NO:2 or an amino acid sequence having at least 95% of sequence identity with SEQ ID NO:2, and LCDR3 as shown in SEQ ID NO:3 or an amino acid sequence having at least 95% of sequence identity with SEQ ID NO:3; and
HCDR1 as shown in SEQ ID NO:10 or an amino acid sequence having at least 95% of sequence identity with SEQ ID NO:10, HCDR2 as shown in SEQ ID NO:11 or an amino acid sequence having at least 95% of sequence identity with SEQ ID NO:11, and HCDR3 as shown in SEQ ID NO:12 or an amino acid sequence having at least 95% of sequence identity with SEQ ID NO:12.

2. The anti-CD47 antibody or the antigen-binding fragment thereof according to claim 1, wherein the antibody comprises at least one of a heavy chain variable region sequence and a light chain variable region sequence, at least a part of at least one of the heavy chain variable region sequence and the light chain variable region sequence is from at least one of a mouse-derived antibody, a humanized antibody, a primate-derived antibody or a mutant thereof.

3. The anti-CD47 antibody or the antigen-binding fragment thereof according to claim 2, wherein the amino acid sequence of the light chain variable region is as shown in any one of SEQ ID NOs:5~9 or a sequence having at least 95% sequence identity with any one of SEQ ID NOs:5~9.

4. The anti-CD47 antibody or the antigen-binding fragment thereof according to claim 2, wherein the amino acid sequence of the heavy chain variable region is as shown in any one of SEQ ID NOs:14~18 or a sequence having at least 95% sequence identity with any one of SEQ ID NOs:14~18.

5. The anti-CD47 antibody or the antigen-binding fragment thereof according to any one of claims 2-4, wherein the amino acid sequence of the light chain variable region is as shown in SEQ ID NO:5, the amino acid sequence of the heavy chain variable region is as shown in SEQ ID NO:14; or the amino acid sequence of the light chain variable region is as shown in SEQ ID NO:6, and the amino acid sequence of the heavy chain variable region is as shown in SEQ ID NO:14; or the amino acid sequence of the light chain variable region is as shown in SEQ ID NO:6, and the amino acid sequence of the heavy chain variable region is as shown in SEQ ID NO:15; or the amino acid sequence of the light chain variable region is as shown in SEQ ID NO:6, and the amino acid sequence of the heavy chain variable region is as shown in SEQ ID NO:16; or the amino acid sequence of the light chain variable region is as shown in SEQ ID NO:6, and the amino acid sequence of the heavy chain variable region is as shown in SEQ ID NO:17; or the amino acid sequence of the light chain variable region is as shown in SEQ ID NO:6, and the amino acid sequence of the heavy chain variable region is as shown in SEQ ID NO:18; or the amino acid sequence of the light chain variable region is as shown in SEQ ID NO:8, and the amino acid sequence of the heavy chain variable region is as shown in SEQ ID NO:14; or the amino acid sequence of the light chain variable region is as shown in SEQ ID NO:9, and the amino acid sequence of the heavy chain variable region is as shown in SEQ ID NO:14; or the amino acid sequence of the light chain variable region is as shown in SEQ ID NO:7, and the amino acid sequence of the heavy chain variable region is as shown in SEQ ID NO:16; or the amino acid sequence of the light chain variable region is as shown in SEQ ID NO:8, and the amino acid sequence of the heavy chain variable region is as shown in SEQ ID NO:16; or the amino acid sequence of the light chain variable region is as shown in SEQ ID NO:9, and the amino acid sequence of the heavy chain variable region is as shown in SEQ ID NO:16; or the amino acid sequence of the light chain variable region is as shown in SEQ ID NO:9, and the amino acid sequence of the heavy chain variable region is as shown in SEQ ID NO:18.

6. The anti-CD47 antibody or the antigen-binding fragment thereof according to any one of claims 1-5, wherein the antibody has a constant region, and the heavy chain constant region is selected from any one of IgGI, IgG2, IgG3, IgG4, IgA, IgM, IgE or IgD; and the light chain constant region is a κ or λ chain.

7. The anti-CD47 antibody or the antigen-binding fragment thereof according to claim 6, wherein the species source of the constant region is selected from mouse, rabbit, sheep, monkey, or human.

8. The anti-CD47 antibody or the antigen-binding fragment thereof according to any one of claims 1-7, wherein the antibody is any one or more of a CDR-transplanted antibody, a multimeric antibody or a bispecific antibody.

9. The anti-CD47 antibody or the antigen-binding fragment thereof according to any one of claims 1-8, wherein the antigen-binding fragment is any one or more of F (ab') 2, Fab, scFv, Fv, and a single-domain antibody.

10. The anti-CD47 antibody or the antigen-binding fragment thereof according to any one of claims 1-9, wherein the CD47 is a human CD47, a mouse CD47 or a monkey CD47.

11. A nucleic acid, wherein the nucleic acid encodes the anti-CD47 antibody or the antigen-binding fragment thereof according to any one of claims 1~10.

12. A vector, wherein the vector comprises the nucleic acid according to claim 11.

13. A cell, wherein the cell comprises the nucleic acid according to claim 11 or the vector according to claim 12.

14. A pharmaceutical composition, wherein the pharmaceutical composition comprises the anti-CD47 antibody or the antigen-binding fragment thereof according to any one of claims 1~10, the nucleic acid according to claim 11, the vector according to claim 12 or the cell according to claim 13.

15. The pharmaceutical composition according to claim 14, wherein the composition further comprises a pharmaceutically acceptable carrier.

16. Use of the anti-CD47 antibody or the antigen-binding fragment thereof according to any one of claims 1~10, the nucleic acid according to claim 11, the vector according to claim 12, the cell according to claim 13 or the composition according to claim 14 in preparation of a drug for treating and/or preventing a CD47-positive tumor.

17. The use according to claim 16, wherein the drug can reduce hemagglutination of red blood cells.

18. The use according to claim 16, wherein the drug can reduce its binding activity to the red blood cells.

19. Use of the anti-CD47 antibody or the antigen-binding fragment thereof according to any one of claims 1~10, the nucleic acid according to claim 11, the vector according to claim 12, or the cell according to claim 13 in preparation of a kit for detecting CD47 or diagnosing a CD47-related disease.

20. A kit for detecting CD47, wherein the kit comprises the anti-CD47 antibody or the antigen-binding fragment thereof according to any one of claims 1~10.

21. A kit for diagnosing a CD47-related disease, wherein the kit comprises the anti-CD47 antibody or the antigen-binding fragment thereof according to any one of claims 1~10.


SEQUENCE LISTING

<110>  GANGDONG FEIPENG PHARMACEUTICAL CO. LTD

<120>  Antibody targeting CD47 and application thereof

<130>  PN182536FPSW

<150>  CN202011544262.6
<151>  2020-12-23

<160>  18

<170>  PatentIn version 3.5

<210>  1
<211>  17
<212>  PRT
<213>  Artificial Sequence

<220>
<223>  Synthesized

<400>  1

Lys Ser Ser Gln Ser Leu Leu Asn Ser Arg Thr Arg Lys Asn Tyr Leu
1               5                   10                  15

Ala

<210>  2
<211>  7
<212>  PRT
<213>  Artificial Sequence

<220>
<223>  Synthesized

<400>  2

Trp Ala Ser Thr Arg Glu Ser
1               5

<210>  3
<211>  8
<212>  PRT
<213>  Artificial Sequence

<220>
<223>  Synthesized

<400>  3

Lys Gln Ser Tyr Asn Leu Arg Thr
1               5

<210>  4
<211>  112

<212>  PRT
<213>  Artificial Sequence

<220>
<223>  Synthesized

<400>  4

Asp Ile Val Met Ser Gln Ser Pro Ser Ser Leu Ala Val Ser Ala Gly
1               5                   10                  15

Glu Lys Val Thr Met Ser Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Lys Gln
                85                  90                  95

Ser Tyr Asn Leu Arg Thr Phe Gly Gly Gly Thr Lys Leu Glu Ile Lys
            100                 105                 110


<210>  5
<211>  112
<212>  PRT
<213>  Artificial Sequence

<220>
<223>  Synthesized

<400>  5

Asp Ile Val Met Thr Gln Ser Pro Asp Ser Leu Ala Val Ser Leu Gly
1               5                   10                  15

Glu Arg Ala Thr Met Asn Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr

65                  70                  75                  80

Ile Ser Ser Leu Gln Ala Glu Asp Val Ala Val Tyr Tyr Cys Lys Gln
                85                  90                  95

Ser Tyr Asn Leu Arg Thr Phe Gly Gly Gly Thr Lys Leu Glu Ile Lys
            100                 105                 110

<210>  6
<211>  112
<212>  PRT
<213>  Artificial Sequence

<220>
<223>  Synthesized

<400>  6

Asp Ile Gln Met Thr Gln Ser Pro Ser Ser Leu Ser Ala Ser Val Gly
1               5                   10                  15

Asp Arg Val Thr Met Thr Cys Lys Ser Ser Gln Ser Leu Leu Asn Ser
            20                  25                  30

Arg Thr Arg Lys Asn Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Lys Gln
                85                  90                  95

Ser Tyr Asn Leu Arg Thr Phe Gly Gly Gly Thr Lys Leu Glu Ile Lys
            100                 105                 110

<210>  7
<211>  112
<212>  PRT
<213>  Artificial Sequence

<220>
<223>  Synthesized

<400>  7

Asp Ile Gln Met Thr Gln Ser Pro Ser Ser Leu Ser Ala Ser Val Gly
1               5                   10                  15

Asp Arg Val Thr Met Thr Cys Lys Ser Ser Gln Ser Leu Leu Asn Thr
            20                  25                  30

Arg Thr Arg Lys Asn Tyr Leu Ala Trp Tyr Gln Gln Lys Pro Gly Lys
        35                  40                  45

Ala Pro Lys Leu Leu Ile Tyr Trp Ala Ser Thr Arg Glu Ser Gly Val
    50                  55                  60

Pro Ser Arg Phe Ser Gly Ser Gly Ser Gly Thr Asp Phe Thr Leu Thr
65                  70                  75                  80

Ile Ser Ser Leu Gln Pro Glu Asp Phe Ala Thr Tyr Tyr Cys Lys Gln
                85                  90                  95

Ser Tyr Asn Leu Arg Thr Phe Gly Gly Gly Thr Lys Leu Glu Ile Lys
            100                 105                 110

<210> 8
<211> 112
<212> PRT
<213> Artificial Sequence

<220>
<223> Synthesized

<400> 8

Asp Ile Val Met Thr Gln Ser Pro Asp Ser Leu Ala Val Ser Leu Gly
1               5                   10                  15

Glu Arg Ala Thr Met Asn Cys Lys Ser Ser Gln Ser Leu Leu Asn Thr
            20                  25                  30

Arg Thr Arg Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Lys Gln
                85                  90                  95

Ser Tyr Asn Leu Arg Thr Phe Gly Gly Gly Thr Lys Leu Glu Ile Lys
            100                 105                 110

<210> 9

<211>  112
<212>  PRT
<213>  Artificial Sequence

<220>
<223>  Synthesized

<400>  9

Glu Ile Val Met Thr Gln Ser Pro Pro Thr Leu Ser Leu Ser Pro Gly
1               5                   10                  15

Glu Arg Val Thr Met Ser Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Ala Pro Arg Leu Leu Ile Tyr Trp Ala Ser Thr Arg Glu Ser Gly Ile
    50                  55                  60

Pro Ala Arg Phe Ser Gly Ser Gly Ser Gly Thr Asp Phe Thr Leu Thr
65                  70                  75                  80

Ile Ser Ser Leu Gln Pro Glu Asp Phe Ala Val Tyr Tyr Cys Lys Gln
                85                  90                  95

Ser Tyr Asn Leu Arg Thr Phe Gly Gly Gly Thr Lys Leu Glu Ile Lys
            100                 105                 110

<210>  10
<211>  5
<212>  PRT
<213>  Artificial Sequence

<220>
<223>  Synthesized

<400>  10

Ser Asn Trp Met Asn
1               5

<210>  11
<211>  17
<212>  PRT
<213>  Artificial Sequence

<220>
<223>  Synthesized

<400>  11

Met Ile His Pro Ser Asp Ser Glu Thr Arg Leu Asn Gln Lys Phe Lys
1               5                   10                  15

Asp

<210> 12
<211> 10
<212> PRT
<213> Artificial Sequence

<220>
<223> Synthesized

<400> 12

Gly Thr Thr Val Val Asp Ala Phe Ala Tyr
1               5                   10

<210> 13
<211> 119
<212> PRT
<213> Artificial Sequence

<220>
<223> Synthesized

<400> 13

Gln Val Gln Leu Gln Gln Pro Gly Ala Glu Leu Val Arg Pro Gly Ala
1               5                   10                  15

Ser Val Lys Leu Ser Cys Lys Ala Ser Gly Ser Ser Phe Thr Ser Asn
            20                  25                  30

Trp Met Asn Trp Val Lys Arg Arg Pro Gly Gln Gly Leu Glu Trp Ile
        35                  40                  45

Gly Met Ile His Pro Ser Asp Ser Glu Thr Arg Leu Asn Gln Lys Phe
    50                  55                  60

Lys Asp Lys Ala Thr Leu Thr Val Asp Lys Ser Ser Ser Thr Ala Tyr
65                  70                  75                  80

Met Gln Leu Ser Ser Pro Thr Ser Glu Asp Ser Ala Val Tyr Tyr Cys
                85                  90                  95

Ala Arg Gly Thr Thr Val Val Asp Ala Phe Ala Tyr Trp Gly Gln Gly
            100                 105                 110

Thr Leu Val Thr Val Ser Ala
        115

<210> 14

<211>  119
<212>  PRT
<213>  Artificial Sequence

<220>
<223>  Synthesized

<400>  14

Gln Val Gln Leu Val Gln Pro Gly Ala Glu Val Lys Lys Pro Gly Ala
1               5                   10                  15

Ser Val Lys Leu Ser Cys Lys Ala Ser Gly Ser Ser Phe Thr Ser Asn
            20                  25                  30

Trp Met Asn Trp Val Arg Gln Ala Pro Gly Gln Gly Leu Glu Trp Ile
        35                  40                  45

Gly Met Ile His Pro Ser Asp Ser Glu Thr Arg Leu Asn Gln Lys Phe
    50                  55                  60

Lys Asp Arg Ala Thr Leu Thr Val Asp Lys Ser Thr Ser Thr Ala Tyr
65                  70                  75                  80

Met Glu Leu Ser Ser Pro Arg Ser Glu Asp Thr Ala Val Tyr Tyr Cys
                85                  90                  95

Ala Arg Gly Thr Thr Val Val Asp Ala Phe Ala Tyr Trp Gly Gln Gly
            100                 105                 110

Thr Thr Val Thr Val Ser Ser
        115

<210>  15
<211>  119
<212>  PRT
<213>  Artificial Sequence

<220>
<223>  Synthesized

<400>  15

Gln Val Gln Leu Val Gln Ser Gly Ala Glu Val Lys Lys Pro Gly Ala
1               5                   10                  15

Ser Val Lys Val Ser Cys Lys Ala Ser Gly Ser Ser Phe Thr Ser Asn
            20                  25                  30

Trp Met Asn Trp Val Arg Gln Ala Pro Gly Gln Gly Leu Glu Trp Ile
        35                  40                  45

Gly Met Ile His Pro Ser Asp Ser Glu Thr Arg Leu Asn Gln Lys Phe
    50                  55                  60

Lys Asp Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Thr Thr Val Val Asp Ala Phe Ala Tyr Trp Gly Gln Gly
            100                 105                 110

Thr Thr Val Thr Val Ser Ser
        115

<210>  16
<211>  119
<212>  PRT
<213>  Artificial Sequence

<220>
<223>  Synthesized

<400>  16

Gln Val Gln Leu Val Gln Ser Gly Ala Glu Val Lys Lys Pro Gly Ser
1               5                   10                  15

Ser Val Lys Val Ser Cys Lys Ala Ser Gly Ser Ser Phe Thr Ser Asn
            20                  25                  30

Trp Met Asn Trp Val Arg Gln Ala Pro Gly Gln Gly Leu Glu Trp Ile
        35                  40                  45

Gly Met Ile His Pro Ser Asp Ser Glu Thr Arg Leu Asn Gln Lys Phe
    50                  55                  60

Lys Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Thr Thr Val Val Asp Ala Phe Ala Tyr Trp Gly Gln Gly
            100                 105                 110

Thr Thr Val Thr Val Ser Ser
        115

<210>  17

<211>  119
<212>  PRT
<213>  Artificial Sequence

<220>
<223>  Synthesized

<400>  17

Gln Val Gln Leu Val Gln Ser Gly Ala Glu Val Lys Lys Pro Gly Ser
1               5                   10                  15

Ser Val Lys Val Ser Cys Lys Ala Ser Gly Ser Ser Phe Thr Ser Asn
            20                  25                  30

Trp Met Asn Trp Val Arg Gln Ala Pro Gly Gln Gly Leu Glu Trp Ile
        35                  40                  45

Gly Met Ile His Pro Ser Asp Ser Glu Thr Arg Leu Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Thr Thr Val Val Asp Ala Phe Ala Tyr Trp Gly Gln Gly
            100                 105                 110

Thr Thr Val Thr Val Ser Ser
        115


<210>  18
<211>  119
<212>  PRT
<213>  Artificial Sequence

<220>
<223>  Synthesized

<400>  18

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Thr Ser Asn
            20                  25                  30

Trp Met Asn Trp Val Arg Gln Ala Pro Gly Gln Gly Leu Glu Trp Ile
        35                  40                  45

Gly Met Ile His Pro Ser Asp Ser Glu Thr Arg Leu Asn Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Thr Thr Val Val Asp Ala Phe Ala Tyr Trp Gly Gln Gly
            100                 105                 110

Thr Thr Val Thr Val Ser Ser
        115